# EUROPEAN PATENT APPLICATION

(11) **EP 4 760 538 A1**
(43) Date of publication of application: **17.06.2026**
(21) Application number: 24219480.1
(22) Date of filing: 12.12.2024
(51) Int. Cl.: G06F 16/58, G06F 16/535

(54) **RESPONDING TO A SEARCH QUERY**

(71) Applicant: Koninklijke Philips N.V., 5656 AE Eindhoven (NL)
(72) Inventor: WILD, Sebastian, Eindhoven (NL); WEBER, Frank Michael, Eindhoven (NL); WAECHTER-STEHLE, Irina, 5656AG Eindhoven (NL); SCHULZ, Heinrich, Eindhoven (NL); PETERS, Jochen, Eindhoven (NL); WEHLE, Simon, Eindhoven (NL)
(74) Representative: Philips Intellectual Property & Standards

(57) **Abstract**

A mechanism for generating a query response to a search query for use in a clinical environment. A conversion module is configured to convert metadata, produced during segmentation mesh modification by a model-based segmentation method, into a text-based file. A search module is configured to process the search query and the text-based file to produce a query response to a user at an output user interface.

## Description

### FIELD OF THE INVENTION

The present invention relates to the field of search queries, and in particular to responding to search queries for information about a medical subject.

### BACKGROUND OF THE INVENTION

There is an increasing use and reliance upon automated or computer-based algorithms and systems within the field of medicine.

As an example, model-based segmentation provides a mechanism for producing a segmentation mesh that represents a representation of an anatomical structure in a medical image. The segmentation mesh may, for instance, provide a clinician or other user with information about the anatomical boundaries of the anatomical structure, and can be further processed to derive measurements or other properties of the anatomical structure.

A typical model-based segmentation algorithm comprises reconfiguring an initial segmentation mesh to match or model the shape of the representation of the anatomical structure within the medical image.

Another area of interest for computer-based systems within the medical field is a system that is able to provide a clinician or other user with accurate and timely responses to user queries relevant for aiding in the making of clinical decisions (e.g., diagnoses or analyses). Such user queries may, for instance, request information about a particular medical subject.

There is therefore a demand for more accurate and reliable mechanisms for responding to user queries.

### SUMMARY OF THE INVENTION

The invention is defined by the claims.

In accordance with a proposed approach, there is provided a user interface system for responding to a search query.

The user interface system comprises a data input interface configured to receive metadata for a segmentation mesh of a representation of an anatomical structure in a medical image, wherein the metadata comprises parameter data generated by a model-based segmentation algorithm during production of the segmentation mesh by reconfiguration of an initial segmentation mesh to the representation of the anatomical structure.

The user interface system further comprises a conversion module configured to convert the metadata into a text-based file containing one or more textual descriptors of the parameter data comprised in the metadata, a user input interface configured to receive the search query from a user, a search module configured to process at least the search query and the text-based file to produce a query response, and a user output interface configured to provide a user-perceptible output representing the query response to the user.

The present disclosure provides an approach for providing a query response to a user, which query response is able to make use of metadata produced during a model-based segmentation of a medical image. In particular, it is herein proposed to provide a mechanism for converting parameter data of such metadata into a text-based file that is more easily processible by a search module.

The proposed system thereby enables more efficient searching and retrieval of information related to medical image segmentation by converting complex metadata into searchable and contextually relevant text. The user input interface and the user output interface allow users to easily query and obtain relevant information about anatomical structures without the need to review or analyze the metadata and/or text-based file directly. This improves the usability and exploitability of imaging data, providing a user with previously unavailable or inaccessible information.

In some embodiments, the search module is configured to process the search query and the text-based file using a first large language model (LLM) algorithm to produce the query response. The use of a large language model algorithm facilitates automated interpretation of complex queries, whilst also being able to make use of the text-based file. Thus, the proposed approach of converting metadata into a text-based file is particularly advantageous when an LLM is used to produce the query response, as the LLM is designed for processing and analyzing textual information. Thus, the text-based file format allows the LLM to exploit its natural language understanding capabilities to extract relevant data and insights from the segmentation metadata. This facilitates more nuanced and contextually appropriate responses to user queries about the anatomical structures represented in the medical images.

In some embodiments, the first large language model algorithm is responsive to one or more system prompts, wherein at least one of the one or more system prompts identifies the text-based file. A system prompt gives instructions to a LLM on how to interpret and use the input given by the user. By identifying the text-based file in the system prompt(s), any need to retrain or further train the LLM is mitigated or avoided, allowing the use of pre-existing LLMs in the proposed system.

In some embodiments, the parameter data comprises a plurality of data elements, each containing a different piece of information generated by the model-based segmentation algorithm during the production of the segmentation mesh, and the conversion module is configured to generate a textual descriptor of each data element to produce the one or more textual descriptors. This approach provides separate conversion of individual data elements into textual descriptors, to provide a more complete (overall) representation of the segmentation process. Moreover, this approach provides a rules or template based approach for producing the textual descriptors, which is computationally efficient.

In some embodiments, the plurality of data elements comprises one or more weights of one or more shape eigenmodes determined by the model-based segmentation algorithm during the production of the segmentation mesh. Including shape eigenmode weights in the metadata (and subsequently produced text-based file) provides the search module with information that provides insights into the geometric characteristics of the segmented anatomical structure, e.g., compared to population averages or the like.

In some embodiments, the plurality of data elements comprises a measure of confidence that a shape of the segmentation mesh matches a shape of the anatomical structure. By incorporating at least one confidence measure in the text-based file, the search module is able to qualify any query response, to reduce a risk of misinterpretation or overreliance by a user on the query response.

In some embodiments, the plurality of data elements comprises a measure of internal energy, being a measure of adherence of the segmentation mesh to the initial segmentation mesh, and/or a measure of external energy, being a measure of adherence of the segmentation mesh to a shape of the representation of the anatomical structure. The inclusion of internal and external energy measures provides valuable information about the segmentation process success and/or deviation from a baseline, which can be indicative of an accuracy of the segmentation and/or an unusualness of the subject.

In some embodiments, the conversion module is configured to process each data element using a respective predefined function for automated generation of the textual descriptor of said data element. The use of predefined functions for descriptor generation ensures consistency and efficiency in the conversion process, as well as reducing a risk of errors in the conversion processing whilst also enabling rapid processing of large volumes of metadata.

In some embodiments, the conversion module is configured to concatenate each textual descriptor together to produce the text-based file. This approach provides a comprehensive text-based representation of the metadata with minimal processing overhead.

In some embodiments, the metadata comprises one or more anatomical measures derived from the segmentation mesh. By including derived anatomical measures, the text-based filed provides the search module with direct access to clinically relevant information. This feature allows the search module to quickly retrieve and analyze specific anatomical characteristics without the need for additional processing.

In some embodiments, the conversion module is configured to convert the metadata into a text-based file using a second large language model. This approach uses a second large language model for metadata conversion, which may function to generate more nuanced and contextually rich textual descriptions.

In some embodiments, the data input interface is further configured to receive measurement data comprising one or more measurements of the anatomical structure produced by a measurement algorithm that processes the segmentation mesh, and the search module is further configured to process the measurement data to produce the query response.

The integration of measurement data into the search process provides the search module with access to quantitative information about the anatomical structures. This facilitates more comprehensive and data-driven query responses, supporting advanced clinical analysis and research applications.

In accordance with another proposed approach, there is provided a system comprising the user interface system as described above and a segmentation system. The segmentation system is configured to receive the medical image comprising the representation of the anatomical structure, receive the initial segmentation mesh, and reconfigure the initial segmentation mesh to the representation of the anatomical structure in the medical image using the model-based segmentation algorithm, to thereby produce the segmentation mesh.

In accordance with another proposed approach, there is provided a computer-implemented method for responding to a search query. The method comprises receiving metadata for a segmentation mesh of a representation of an anatomical structure in a medical image, wherein the metadata comprises parameter data generated by a model-based segmentation algorithm during production of the segmentation mesh by reconfiguration of an initial segmentation mesh to the representation of the anatomical structure. The method further comprises converting the metadata into a text-based file containing one or more textual descriptors of the parameter data comprised in the metadata, receiving the search query from a user, processing at least the search query and the text-based file to produce a query response, and controlling a user output interface to provide a user-perceptible output representing the query response to the user.

The skilled person would be readily capable of modifying the computer-implemented method to perform the function(s) of any herein disclosed system or user interface system, and vice versa.

In accordance with yet another proposed approach, there is provided a computer program product comprising computer program code means which, when executed on a computing device having a processing system, cause the processing system to perform all of the steps of the computer-implemented method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an existing user interface system;
Fig. 2 illustrates a proposed user interface system; and
Fig. 3 is a flowchart illustrating a proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating a query response to a search query for use in a clinical environment. A conversion module is configured to convert metadata, produced during segmentation mesh modification by a model-based segmentation method, into a text-based file. A search module is configured to process the search query and the text-based file to produce a query response to a user at an output user interface.

In the context of the present disclosure, a medical image is any digital image of a subject produced using a medical imaging modality, technique and/or system, such as an ultrasound image, a PET image, a CT image, an MR image, an X-ray image and so on. Preferably, the medical image comprises a representation of at least one internal structure or internal physiological process of the subject. The medical image may be a two-dimensional medial image, a three-dimensional medical image or a two- or three-dimensional image for a sequence of points in time (i.e., a time series of 2D medical images)

In the context of the present disclosure, a segmentation mesh may be defined as a collection of interconnected vertices and edges that form a representation of the surface or boundary of an anatomical structure. The segmentation mesh may be digital or computationally represented as a set of coordinates for the vertices and a list of connections between these vertices, defining the edges and faces of the mesh. Typically, a segmentation mesh will define a plurality of triangles that form the faces of the mesh, although other example shapes are known in the art.

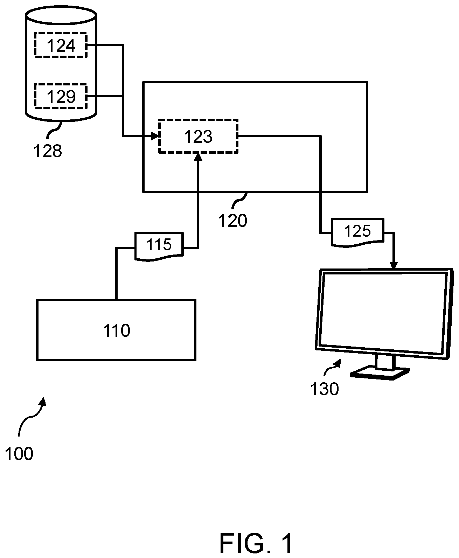

Fig. 1 illustrates an existing user interface system 100, for the purposes of improved contextual understanding. The user interface system 100 comprises a user input interface 110; a search module 120; and a user output interface 130.

The search module 120 is designed for processing and responding to search queries related to medical subjects. The user input interface 110 and user output interface 120 are designed for facilitating interaction between a user and the search module for processing search queries related to medical subjects.

In particular, the user input interface 110 is configured to receive a search query 115, e.g., about the medical subject. The user input interface 110 may, for instance, comprise a text input device (e.g., a keyboard or the like), a voice recognition system (e.g., with voice- to-text conversion capabilities) and/or an interactive graphical user interface (e.g., a touchscreen or similar). As such, the search query may comprise textual data for processing by the search module.

The search module 120 is configured to process the search query 115 to produce a query response 125.

In general terms, the search module 120 may be configured to process at least the search query 115 and a dataset 129 to produce a query response 125. Thus, the search module comprises at least one processor configured to process the search query 115 and the dataset 129 to produce the query response 125.

More particularly, the search module 120 may be configured to interpret the search query 115, search through the relevant information in the dataset 129, and formulate a suitable query response 125.

The dataset 129 carries or contains searchable medical information and/or knowledge bases. For instance, the dataset may comprise clinical data (for a plurality of subjects), research findings, treatment protocols, and/or diagnostic information (e.g., for a plurality of subjects). The dataset 129 may be stored in a memory 128 communicatively coupled to the search module.

Approaches for processing a search query 115 in this manner to produce a query response are widely known in the art.

By way of example, the search module may use a large-language model 123 (e.g., running on at least one processor of the search module) to process the search query 115 and the dataset 129 to produce the query response 125. Known examples of large-language models 123 are also configured to be responsive to one or more systems prompts 124, which define or control a desired behavior of the large-language model 123. The system prompt(s) 124 may be stored in a/the memory 128, communicatively coupled to the search module.

Suitable examples of a system prompts include text prompts such as: "Be as precise as possible in your answers and avoid formulating too long sentences" or "Use your preexisting knowledge to deduce the experience level of the user from his or her question. Formulate your answer such that its level of detail matches this experience level". Other examples will be readily apparent to the skilled person.

Suitable examples of a large-language model include those set out in Thirunavukarasu, Arun James, et al. "Large language models in medicine." Nature medicine 29.8 (2023): 1930-1940 or Zhao, Wayne Xin, et al. "A survey of large language models." arXiv preprint arXiv:2303.18223 (2023).

In practice, if used, the large-language model 123 is stored in a memory or storage unit, such as a cloud-based storage system. Similarly, the search module 120 may itself be embodied in a cloud-based processing system.

Alternative approaches and methods for processing a search query 115 to produce a query response 125 are known, such as rule-based systems, keyword matching algorithms, and natural language processing techniques (e.g., employing one or more semantic analysis methods). In general, these approaches comprise parsing the search query, identifying relevant keywords or concepts, and retrieving information from structured databases or knowledge bases to produce a query response.

The user output interface 130 is configured to provide a user-perceptible output responsive to the query response, e.g., representing the query response. In other words, the user output interface 130 is configured to present the query response to the user in a user-perceptible format.

The user-perceptible output may, for instance, comprise a visual representation or an audio representation. Thus, the user output interface 130 may comprise a screen/display configured to provide a visual representation (e.g., text on a screen) of the query response. In some examples, the user output interface 130 comprises a speaker for providing an audio representation of the query response. Other suitable examples will be readily apparent to the skilled person.

The proposed system provides an improved mechanism for responding to search queries. In particular, the proposed system makes previously unusable, but medically relevant, information available to the search module for improving the ability of the search module to accurately respond to the search query. This directly provides the user with previously unavailable information, for improved clinical understanding and aiding in clinical decision making.

The present disclosure recognizes that the production of a segmentation mesh (which represents an anatomical structure) for a medical image using a model-based segmentation algorithm inherently creates parameter data.

It has been recognized that this parameter data contains valuable information for facilitating improved understanding of the anatomical model and the segmentation mesh, which would aid a clinician in making a clinical decision (e.g., diagnosing or otherwise assessing the subject associated with the anatomical structure).

However, it has also been identified that the information contained in the parameter data is difficult to interpret, and is typically structured in a format that makes it difficult for a search module to process.

In other words, exposing this information to a user (such as a clinician) in its "raw" form, e.g. via log files or a table containing the parameter data is not feasible. The overall amount of internal information generated by a model-based segmentation algorithm is huge, and it is neither practical nor realistic for a user to manually inspect the corresponding log files or tables.

The present disclosure provides a mechanism for overcoming at least some of these problems.

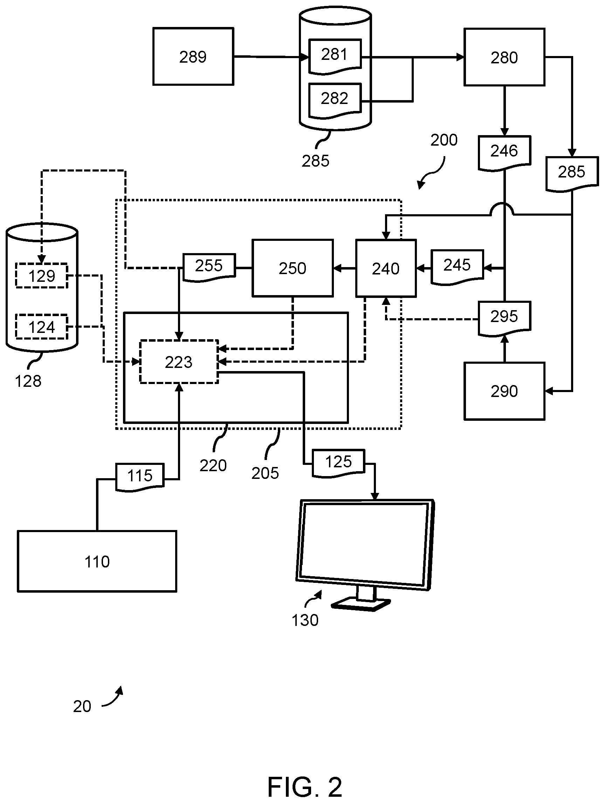

Fig. 2 illustrates a proposed user interface system 200, which again comprises a user input interface 110 and a user output interface 130, which may be embodied as previously disclosed.

The user interface system further comprises a data input interface 240; a conversion module 250; and a search module 220. In practice, the data input interface 240, the conversion module 250 and the search module 220 may form different aspects or parts of a same shared processing system 205.

The data input interface 240 is configured to receive metadata 245 for a segmentation mesh of a representation of an anatomical structure in a medical image. The metadata 245 comprises parameter data 246 generated by a model-based segmentation algorithm during production of the segmentation mesh 285. As later described in detail, production of the segmentation mesh takes place by reconfiguration of an initial segmentation mesh to the representation of the anatomical structure.

It is noted that the parameter data may be generated and output concurrently with the production of the segmentation mesh, rather than being derived from post-processing of the final segmentation mesh. In particular, during the model-based segmentation process, the algorithm may track and record various parameters (examples of which are later provided) as it iteratively refines the initial segmentation mesh to match the anatomical structure in the medical image. These parameters may include intermediate shape variations, confidence measures, energy metrics, and other relevant data points that describe the progression and characteristics of the segmentation process.

As the algorithm converges on the final segmentation mesh, it may output both the segmentation mesh 285 itself and the accumulated parameter data 246 as separate but related outputs. In this way, the metadata 245 may comprise valuable information about the segmentation process that may not be directly inferable from the final mesh alone.

In some examples, as illustrated, the parameter data 246 of the metadata 245 may be generated by and retrieved (by the data input interface 240) from a segmentation system 280 that performs the model-based segmentation algorithm to produce the segmentation mesh. This segmentation system may be integrated with or separate from the user interface system. Thus, the metadata may be directly retrieved by the data input interface as part of a real-time or near real-time processing pipeline.

In other cases, the metadata may be stored in a memory after being generated by the segmentation process, and later retrieved by the data input interface 240. The memory may, for instance, comprise a cloud-based storage system or distributed computing environment.

In some approaches, as later described, the metadata may be received from multiple sources and combined by the data input interface. For example, some parameter data may come directly from the segmentation system or memory, while other metadata elements like anatomical measurements 290 may be generated by a separate system.

The skilled person will readily appreciate how the data input interface 240 may be embodied to facilitate the reception of metadata for the segmentation mesh. For instance, the data input interface 240 may comprise a network interface configured to receive data over a communication network, such as a local area network (LAN) or wide area network (WAN). In some examples, the data input interface may comprise a direct hardware connection, such as a high-speed data bus or serial interface, for receiving metadata directly from an integrated segmentation system.

As previously explained, performing model-based segmentation of an anatomical structure in a medical image produces parameter data, examples of which are provided later in this disclosure. Moreover, as later described and exemplified, the metadata 245 (and the segmentation mesh 285) may be produced by a segmentation system 280 that processes the medical image 282 using the model-based segmentation algorithm to produce the metadata 245.

The conversion module 250 is configured to convert the metadata 245 into a text-based file 255 containing one or more textual descriptors of the parameter data comprised in the metadata. Example configurations for the conversion module 250 are provided later.

The search module 220 is configured to process (at least) the search query 115 (provided by the user input interface 110) and the text-based file 255 to produce the query response 125. In particular, the search module may search the text-based file for data or textual descriptors that are semantically relevant to the search query 115 and prepare a query response 125 based on any identified textual descriptors.

The search module 220 may comprise one or more processors configured to perform the desired functionality of the search module, e.g., when running instructions stored by a memory of the search module.

In some examples, in a similar manner to that previously disclosed, the search module is configured to process the search query 115 and the text-based file 255 using a first large language model algorithm 223 to produce the query response 125. Other approaches for processing a user query and text-based file to produce a query response are well known to the skilled person, some examples of which have been previously mentioned (e.g., rule-based systems, keyword matching algorithms, and natural language processing techniques).

The proposed technique is particularly advantageous when using a first large language model algorithm, as such algorithms are adapted or designed for the processing of textual information. By structuring the metadata in the form of a text-based file, the data is restricted in a format that is more efficient and/or directly designed for processing by the large-language model.

From the foregoing, it will be apparent that the text-based file effectively functions as a bridge between the technical metadata produced by a model-based segmentation method and the natural language processing capabilities of the large language model. This facilitates more comprehensive and insightful responses to search queries.

In some examples, the search module is configured to process the search query and a dataset 129 to produce the query response. In such examples, the dataset may comprise at least the text-based file, and optionally other pre-existing data or knowledge bases (e.g., other medical information of medical subjects or the like), such as that described with reference to the existing user interface system 100 (Fig. 1).

In such examples, the conversion module need not directly provide the text-based file to the search module 220 (as illustrated). Rather, the text-based file may be stored as part of the dataset 129 that the search module processes.

As previously mentioned, the dataset 129 may comprise (in addition to the text-based file) a wide range of medical information, such as clinical data, research findings, treatment protocols, and diagnostic information for various subjects. By incorporating this additional data, the search module is able to provide more contextually rich and informative responses to user queries.

In some examples, the search module is configured to also process the segmentation mesh (or further information derived from the segmentation mesh) in producing the query response.

By way of example, the data input interface may be configured to receive the segmentation mesh and the conversion module may be configured to convert the segmentation mesh into a second text-based file, e.g., in a data format suitable for processing by the search module. This second text-based file may be provided to the search module 220 or form part of the dataset 129.

The conversion module may, for instance, be configured to convert segmentation mesh into a text-based format by encoding the segmentation mesh's vertices, edges, and faces as structured text data. This might include information about vertex coordinates, connectivity between vertices, and surface normal vectors. Such a representation would allow the search module to perform geometric calculations or pattern recognition directly on the mesh data when formulating responses to user queries.

Furthermore, this capability enables the search module to correlate the textual descriptors derived from the metadata with the actual geometric properties of the segmentation mesh. This correlation can lead to more contextually rich and clinically relevant query responses, as the system can now draw insights from both the parametric data generated during the segmentation process and the final geometric representation of the anatomical structure.

In embodiments in which the search module 220 is configured to use a first large language model to produce the query response 125, the first large language model may be responsive to one or more system prompts 124. In general, a system prompt gives instructions to a trained LLM on how to interpret and use an input (here: the search query) given by the user.

Preferably, in such examples, at least one of the one or more system prompts identifies the text-based file, e.g., identifies a location of the text-based file. This facilitates the simple integration or instruction of a known or pre-trained large-language model for use with the proposed system, and in particular for using the text-based file.

In particular, the identification of the text-based file within the system prompt(s) serves to direct the first large language model's attention to the converted metadata, ensuring that the model incorporates this valuable information when processing the search query. This approach allows the first large-language model to seamlessly integrate the segmentation-derived metadata with its broader knowledge base, improving its ability to provide clinically meaningful answers to user queries.

By way of example, an example system prompt may be "The user will ask you questions about the internal parameters of model-based segmentation of an Ultrasound image. Those internal parameters have been determined by the algorithm, and are summarized in this text file: [GENERATED LOG FILE]", where the placeholder [GENERATED LOG FILE] is replaced by an identification of the (location of the) text-based file.

It has previously been explained how the conversion module 250 is configured to convert the metadata 245 into the text-based file containing one or more textual descriptors.

The text-based file may be in any suitable text-based formats such as plain text (.txt), rich text format (.rtf), extensible markup language (.xml), JavaScript Object Notation (.json) and/or YAML Ain't Markup Language (.yaml). The choice of format may depend on the specific requirements and configuration of the search module and the nature of the textual descriptors.

In one example, the conversion module 250 is configured to convert the metadata into a text-based file using a second large language model.

This second large language model may take as input at least the raw metadata 246 produced by the model-based segmentation algorithm during production of the segmentation mesh 285. The second large language model produces, as output, the text-based file - such as a "report-like" text containing relevant information.

The second large language model may be a specifically trained large-language model, e.g., a model trained using a training dataset comprising input-output pairs. Each input-output pair comprises an instance of image data (comprising a respective instance of metadata) and a text-based file (e.g., a manually defined or human written text-based file). More specifically, each text-based file may be a manually defined example text prepared by one or more appropriately trained clinicians or users reviewing the corresponding instance of metadata and/or the segmentations meshes produced when said example instance of metadata was generated.

Approaches for training a large language model using example input data and corresponding example output data are well known in the art. These approaches typically make use of one or more supervised learning techniques, in which the model is trained on a training dataset of input-output pairs.

As briefly mentioned above, for the second large language model used in the conversion module, the training dataset would comprise input-output pairs comprising example metadata from model-based segmentation algorithms as inputs, paired with corresponding human-written textual descriptions as outputs. One example training process may comprise iteratively adjusting the model parameters to minimize the difference between its predicted outputs and the actual human-written descriptions. Common techniques include backpropagation, gradient descent, and various optimization algorithms. The trained model is thereafter able to generalize from these examples to convert new, unseen metadata into coherent textual descriptions.

In some examples, the second large language model is further configured to take, as input when producing the text-based file, the segmentation mesh 285 produced by the model-based segmentation algorithm. Accordingly, the data input interface 240 may receive the segmentation mesh 285 and pass this to the conversion module 250 for processing by the second large-language model.

Of course, during any training of the second large language model, the training dataset for the second large language model may be adapted to include a segmentation mesh for each instance of metadata. More specifically, the input-output pairs in the training dataset may be expanded. Each input may now comprise both the metadata and the corresponding segmentation mesh, while the output remains the human-written textual description.
In another approach, the parameter data 246 comprises a plurality of data elements, each containing a different piece of information generated by the model-based segmentation algorithm during the production of the segmentation mesh.

In such an approach, the conversion module 250 may be configured to generate a textual descriptor of each data element to produce the one or more textual descriptors. This approach thereby produces a text snippet (i.e., textual descriptor) for each data element.

The textual descriptors may be concatenated together to produce the text-based file. For instance, the textual descriptors could be simply appended one after another, optionally separated by line breaks or paragraph markers. Alternatively, a more structured approach might be employed, where descriptors are grouped by categories and labeled accordingly within the text-based file.

In some examples, the conversion module is configured to process each data element using a large-language model, to transform the data element into a suitable text snippet. This avoids the need for using explicit templates.

In some examples, the conversion module 250 may be configured to process each data element using a respective predefined function for automated generation of the textual descriptor of said data element. Each predefined function may, for instance, be manually defined (e.g., human-defined).

By way of example, the predefined function may define one or more template textual descriptors for each data element. For each data element, an appropriate template textual description may be identified and completed or filled out using the piece of information contained by the respective data element.

A number of example data elements that parameter data may comprise are later described. However, for improved contextual understanding of the metadata, a brief description of model-based segmentation is hereafter provided.

A model-based segmentation method comprises receiving a medical image (expected to contain a representation of an anatomical structure) and an initial segmentation mesh, also known as a mesh model. The initial segmentation mesh may be a three-dimensional or two-dimensional mesh model representing a desired anatomical structure, the number of dimensions depending upon the number of dimensions of the medical image.

The initial segmentation mesh is modified or adjusted to match the shape of the representation of the anatomical structure in the medical image. This process typically involves iteratively deforming the initial segmentation mesh to align with the boundaries or features of the anatomical structure visible in the medical image. The resulting modified mesh, which closely conforms to the shape of the anatomical structure, is the (final) segmentation mesh.

Example approaches for performing model-based segmentation of anatomical structures are disclosed by, inter alia, Kelemen, András, Gábor Székely, and Guido Gerig. "Elastic model-based segmentation of 3-D neuroradiological data sets." IEEE Transactions on medical imaging 18.10 (1999): 828-839; Brosch, Tom, et al. "Model-based segmentation using neural network-based boundary detectors: Application to prostate and heart segmentation in MR images." Machine Learning with Applications 6 (2021): 100078; and Kaus, Michael R., et al. "Automated 3-D PDM construction from segmented images using deformable models." IEEE Transactions on Medical Imaging 22.8 (2003): 1005-1013.d

During the segmentation process, the algorithm generates parameter data that describes various aspects of the mesh adaptation. This parameter data may include information about shape variations, confidence measures, and energy metrics that quantify how well the mesh adheres to the image data and maintains its original form. In particular, the parameter data may comprise working information used during the modification of the initial segmentation mesh to produce the (final) segmentation mesh. Thus, the parameter data may capture or represent transient or intermediate information that could be lost if only the final segmentation mesh were analyzed.

Thus, the parameter data may include details about the deformation path taken by the mesh, the number of iterations required for convergence, or the relative influence of different shape modes during the adaptation process.

The data elements forming the parameter data may comprise one or more pieces of this parameter data. Examples of data elements are hereafter described.

In some examples, the plurality of data elements comprises one or more weights of one or more shape eigenmodes determined by the model-based segmentation algorithm during the production of the segmentation mesh.

These shape eigenmodes reflect variations of the (patient-specific) shape of the anatomical structure with respect to a population-averaged mean. The shape eigenmodes thereby represent the ways in which the shape of an anatomical structure can vary across a population.

In more detail, shape eigenmodes are derived from statistical analysis of a large number of segmentation meshes for population of subjects. Each shape eigenmode corresponds to a specific pattern of shape variation observable in the population. The weights associated with these shape eigenmodes (during performance of a subsequent model-based segmentation) function to quantify how much of each variation pattern is present in the specific anatomical structure undergoing segmentation.

By way of example only, generating the segmentation mesh for a model-based segmentation of a heart may identify (shape) eigenmodes that capture dilated vs. normal ventricular shapes or subjects with unusually large or small atria. An example of such a model-based segmentation of the heart is the Philips HeartModel.

The inclusion of these eigenmode weights in the parameter data (and subsequently converted for inclusion in the text-based file) is particularly valuable for clinical interpretation, as they provide quantitative measures of how a patient's anatomy compares to population norms. For example, a high weight for an eigenmode associated with ventricular dilation could be an indicator of certain cardiac conditions.

Where the conversion model is configured to process each data element using a respective predefined function to generate the textual descriptor of said data element, then an exemplary text snippet that may be produced by processing an eigenmode weight is as follows: "For the current image, eigenmode #3 which captures the difference between normal and dilated RV shapes has a final weight of 0.23. This indicates a slightly dilated RV, which is within the 83.2% percentile of the distribution within the training set."

Such a text snippet may be produced from a template as follows: "For the current image, eigenmode #3 which captures the difference between normal and dilated RV shapes has a final weight of FINALWEIGHT. This indicates a WEIGHTRESULT, which is within the WEIGHTPERCENTILE percentile of the distribution within the training set.". In this template, capitalized words indicate placeholders that are replaced by appropriate values.

The value of FINAL WEIGHT may be defined by the eigenmode weight of eigenmode 3 (which captures the difference between normal and dilated RV shapes).

The value of WEIGHTRESULT may be determined by processing the eigenmode weight using one or more predetermined rules. The predetermined rules could be defined based on statistical analysis of a large population dataset. These rules may associate each of a plurality of different ranges with different outcomes. For example, the rules might define that an eigenmode weight falling within a certain range corresponds to a "highly dilated" outcome, while another range might indicate a "slightly dilated" outcome, and yet another range could be associated with a "normal" outcome. This approach functions to translate the numerical value into a clinically meaningful description suited for a textual descriptor.

The value of the WEIGHTPERCENTILE may be determined by mapping the eigenmode weight to a percentile value based on a known distribution of eigenmode weights within a training dataset or population

The skilled person will appreciate that the above exemplary text snippet (i.e., textual descriptor) and template are merely exemplary, and that the skilled person would be readily capable of defining or determining other suitable examples for other forms of data element and/or other eigenmode weights.

For instance, the conversion module may process the data element using a large-language model to produce a respective text snippet.

In some examples, the plurality of data elements comprises a measure of confidence that a shape of the segmentation mesh matches a shape of the anatomical structure. It is common for a model-based segmentation method to produce a measure of confidence in the accuracy of the segmentation mesh, which is here exploited to improve the accuracy and reliability of the query response.

Including the measure of confidence in the text-based file allows for generation of more accurate query responses, e.g., to indicate or clarify a certainty of the query response based on the measure of confidence. For instance, the search module (e.g., a large-language model employed by the search model) may use this information to qualify its responses, providing more definitive answers when the confidence is high and more cautious or tentative responses when the confidence is lower.

In some examples, the plurality of data elements comprises a measure of internal energy, being a measure of adherence of the segmentation mesh to the initial segmentation mesh; and/or a measure of external energy, being a measure of adherence of the segmentation mesh to a shape of the representation of the anatomical structure.

One example of a segmentation method that produces measures of internal and external energy is disclosed in Kaus, Michael R., et al. "Automated 3-D PDM construction from segmented images using deformable models." IEEE Transactions on Medical Imaging 22.8 (2003): 1005-1013. Other examples will be well known to the skilled person.

The measure(s) of internal and/or external energy may be defined on a mesh-wide scale or on a local scale (e.g., per: vertex of the mesh, face of the mesh or other sub-region of the mesh).

Thus, the plurality of data elements may comprise a plurality of measures of internal energy, each being a measure of adherence of a respective portion (e.g., vertex, face or other sub-region) of the segmentation mesh to a corresponding portion of the initial segmentation mesh. This local internal energy measure quantifies how much each part of the final segmentation mesh deviates from its corresponding part in the initial mesh, providing insight into which areas of the anatomical structure required more significant adjustments during the segmentation process (i.e., and thereby which areas deviate more from a normal or average shape).

Similarly, the plurality of data elements may comprise a plurality of measures of external energy, each being a measure of adherence of a respective portion (e.g., vertex, face or other sub-region) of the segmentation mesh to a corresponding portion of the representation of the anatomical structure in the medical image. These local external energy measures indicate how well each part of the segmentation mesh aligns with the actual image data, highlighting areas where the segmentation may be more or less accurate.

Including these local energy measures in the metadata allows for a more detailed analysis of the segmentation quality. When converted into the text-based file, these measures can provide valuable information about specific regions of the anatomical structure that may be of particular interest or concern.

For example, a textual descriptor generated from these local energy measures might read: "The segmentation shows high adherence to the image data in the upper left region of the structure, with an average external energy of 0.92, while the lower right region shows lower adherence with an average external energy of 0.78, suggesting potential areas for closer examination."

In addition to vertices and faces, other example sub-regions of the segmentation mesh include edges, patches (i.e., groups of adjacent faces that form a continuous surface), and/or anatomical landmarks or regions of interest identified within the mesh. The sub-regions may be defined based on geometric properties, anatomical significance, or specific features of interest for the particular medical application.

In some examples, the sub-regions may be predefined as part of the initial segmentation mesh, while in other cases, they may be dynamically identified during the segmentation process based on image features or model characteristics.

In some examples, the metadata 245, in addition to the parameter data 246, also comprises one or more anatomical measures 295 derived from the segmentation mesh. The one or more anatomical measures 295 may be produced by a measuring system 290 that uses a measurement algorithm to process the segmentation mesh 285 to produce the one or more anatomical measurements.

Approaches for processing a segmentation mesh to produce one or more anatomical measurements are well known in the art.

The anatomical measures provide quantitative information about the structure and dimensions (i.e., geometric measurements) of the segmented anatomical feature and/or simulated physiological parameters from the segmentation mesh. Example anatomical measures include predicted flow rates, anatomical distances, anatomical radii, anatomical diameters, predicted volumes and so on.

For example, in the case of cardiac segmentation, anatomical measures may include left ventricular volume, ejection fraction, myocardial mass, or wall thickness. For lung segmentation, measures may include lung volume, airway diameter, or nodule size. These measures are typically clinically relevant metrics that aid in diagnosis, treatment planning, or monitoring disease progression.

The anatomical measures 295 may be produced by a separate measuring system 290 that applies specific measurement algorithms to the segmentation mesh 285. This measuring system analyzes the geometry of the mesh to extract meaningful anatomical information. The measurement algorithms may vary depending on the anatomical structure being analyzed and the specific clinical metrics required.

In some cases, the conversion of anatomical measures to textual descriptors may be performed using predefined templates and rules (e.g., if-then rules). This approach may allow for the generation of clinically meaningful descriptions from numerical measurements.

By way of working example, consider a scenario in which the anatomical structure is the heart, and the anatomical measure(s) include a measure of left ventricular volume. In such an example, the following template may be used: "The left ventricular end-diastolic volume is VOLUME ml, which is COMPARISON to the normal range of 60-120 ml for adults." The VOLUME placeholder would be filled with the actual measured value. The COMPARISON placeholder might be filled based on if-then rules such as: if volume < 60 ml, then "below"; If 60 ml ≤ volume ≤ 120 ml, then "within"; if volume > 120 ml, then "above".

In some examples, the interface is further configured to receive measurement data comprising one or more (anatomical) measurements of the anatomical structure produced by a measurement algorithm that processes the segmentation mesh. Example anatomical measures have been previously mentioned.

In such examples, the search module may be further configured to process the measurement data to produce the query response.

In this way, the anatomical measurement(s) may be treated separately or independently of the metadata, thus, the anatomical measurement(s) need not be converted into a text-based format for use in the text-based file.

This approach provides flexibility in how different types of information are processed and utilized within the system. By keeping the anatomical measurements independent, the search module is able to process them in their native numerical format, which may be more efficient for certain types of analysis or queries. Furthermore, maintaining the anatomical measurements in their original format preserves precision that might be lost in a text-based description.

Fig. 2 also illustrates a system 20 comprising the proposed user interface system 200.

The system 200 further comprises a segmentation system 280 configured to receive the medical image 281 comprising the representation of the anatomical structure; receive the initial segmentation mesh 282; and reconfigure the initial segmentation mesh to the representation of the anatomical structure in the medical image using the model-based segmentation algorithm, to thereby produce the segmentation mesh.

The medical image 281 and/or initial segmentation mesh 282 may, as illustrated, be stored in a memory 283 communicatively coupled to the segmentation system 280.

In some examples, the medical image 281 may be retrieved from a medical imaging system 289, such as an MRI, CT, ultrasound, or X-ray machine. This arrangement allows the segmentation system 280 to access the necessary input data for performing the model-based segmentation algorithm. The medical imaging system may be directly connected to the segmentation system 280 or may transmit the medical image 281 over a network to be stored in the memory 283.

The initial segmentation mesh 282, which serves as the starting point for the model-based segmentation algorithm, may be pre-stored in the memory 283.

Thus, the memory 283 may be a storage device capable of storing digital data, including medical images 281 and segmentation meshes 282. It may be implemented as RAM, solid-state drives, or other storage technologies, and may be integrated within or externally connected to the segmentation system 280.

In some examples, the segmentation system may be configured to dynamically identify sub-regions during the model-based segmentation process, e.g., for use in defining at least some parameter data (e.g., internal/external measures of energy). For instance, the segmentation system may analyze local curvature or other geometric properties of the evolving mesh to identify areas of high curvature or rapid shape changes. These areas may be designated as sub-regions of interest.

In some examples, the segmentation system may be configured to output a structured metadata package that includes both the final segmentation mesh and the associated parameter data.

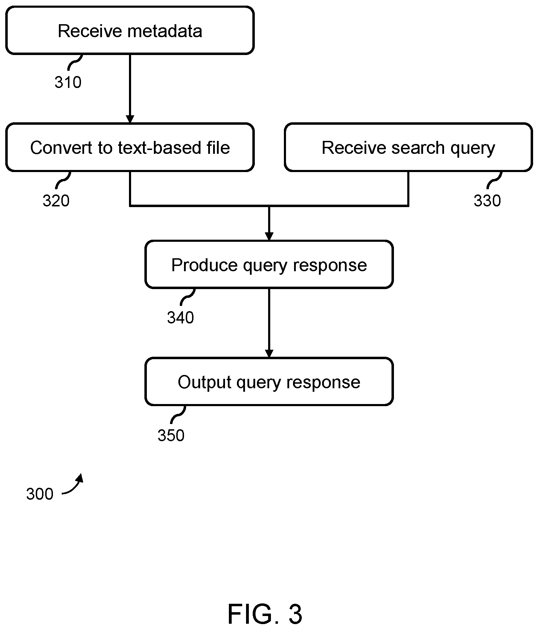

For the sake of completeness, Fig. 3 illustrates a computer-implemented method 300 for responding to a search query, which may be carried out by the previously described user interface system or another processing system/arrangement.

The computer-implemented method 300 comprises a step 310 of receiving metadata for a segmentation mesh of a representation of an anatomical structure in a medical image. The metadata comprises parameter data generated by a model-based segmentation algorithm during production of the segmentation mesh by reconfiguration of an initial segmentation mesh to the representation of the anatomical structure.

Step 310 may, for instance, be performed by a data input interface of the user interface system.

The computer-implemented method 300 also comprises a step 320 of converting the metadata into a text-based file containing one or more textual descriptors of the parameter data comprised in the metadata. Step 320 may be performed by the conversion module previously described.

Approaches for performing step 320 have been previously disclosed, e.g., at least in the context of describing the functionality of the conversion module. Step 320 may be readily adapted for performing any herein proposed functionality of any herein proposed conversion module.

The computer-implemented method 300 also comprises a step 330 of receiving the search query from a user. The search query may be input by the user over a user input interface, as previously described. The search query may, for instance, be textual or text-based query.

The computer-implemented method 300 also comprises a step 340 of processing at least the search query and the text-based file to produce a query response. Step 320 may be readily adapted for performing any herein proposed functionality of any herein proposed search module, e.g., using a large-language model or similar.

The computer-implemented method 300 also comprises a step 350 of controlling a user output interface to provide a user-perceptible output representing the query response to the user. Example user-perceptible outputs have been previously disclosed.

Some proposed embodiments make use of a conversion module to convert the metadata into a text-based file. The conversion module may be embodied as an aspect of a processing system.

Some proposed embodiments make use of a search module to process at least the search query and the text-based file to produce a query response. The search module may be embodied as an aspect of a processing system, e.g., the same processing system comprising the conversion module.

Moreover, the skilled person would also be readily capable of developing a processing system for carrying out any herein described method. Thus, each step of the flow chart may represent a different action performed by a processing system, and may be performed by a respective module of the processing system.

One or more embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

Thus, the processing system may comprise processing system components (e.g., one or more processors) and one or more storage medica carrying instructions that, when executed by the processing system components, cause the processing system components to perform the functions of any herein disclosed method.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

Ordinal numbers (e.g. "first", "second" and so on) have been used purely to distinguish different elements from one another for the sake of clarity, and does not necessarily imply a specific order, importance, relationship, or presence of all numbered elements. Reference to a non-"first" (e.g. "second" or "third") element does not necessitate that a "first" element be present. The skilled person would be capable of relabeling any such elements as appropriate (e.g. relabeling a "second" element as a "first" element if only the second element is present).

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A user interface system for responding to a search query, the user interface system comprising:
a data input interface configured to receive metadata for a segmentation mesh of a representation of an anatomical structure in a medical image, wherein the metadata comprises parameter data generated by a model-based segmentation algorithm during production of the segmentation mesh by reconfiguration of an initial segmentation mesh to the representation of the anatomical structure;
a conversion module configured to convert the metadata into a text-based file containing one or more textual descriptors of the parameter data comprised in the metadata;
a user input interface configured to receive the search query from a user;
a search module configured to process at least the search query and the text-based file to produce a query response; and
a user output interface configured to provide a user-perceptible output representing the query response to the user.

2. The user interface system of claim 1, wherein the search module is configured to process the search query and the text-based file using a first large language model algorithm to produce the query response.

3. The user interface system of claim 2, wherein the first large language model algorithm is responsive to one or more system prompts, wherein at least one of the one or more system prompts identifies the text-based file.

4. The user interface system of any one of claim 1 to 3, wherein:
the parameter data comprises a plurality of data elements, each containing a different piece of information generated by the model-based segmentation algorithm during the production of the segmentation mesh; and
the conversion module is configured to generate a textual descriptor of each data element to produce the one or more textual descriptors.

5. The user interface system of claim 4, wherein the plurality of data elements comprises one or more weights of one or more shape eigenmodes determined by the model-based segmentation algorithm during the production of the segmentation mesh.

6. The user interface system of claim 4 or 5, wherein the plurality of data elements comprises a measure of confidence that a shape of the segmentation mesh matches a shape of the anatomical structure.

7. The user interface system of any one of claims 4 to 6, wherein the plurality of data elements comprises:
a measure of internal energy, being a measure of adherence of the segmentation mesh to the initial segmentation mesh; and/or
a measure of external energy, being a measure of adherence of the segmentation mesh to a shape of the representation of the anatomical structure.

8. The user interface system of any one of claims 4 to 7, wherein the conversion module is configured to process each data element using a respective predefined function for automated generation of the textual descriptor of said data element.

9. The user interface system of any one of claims 4 to 8, wherein the conversion module is configured to concatenate each textual descriptor together to produce the text-based file.

10. The user interface system of any one of claims 1 to 9, wherein the metadata further comprises one or more anatomical measures derived from the segmentation mesh.

11. The user interface system of any one of claims 1 or 2, wherein the conversion module is configured to convert the metadata into a text-based file using a second large language model.

12. The user interface system of any one of claims 1 to 11, wherein:
the data input interface is further configured to receive measurement data comprising one or more measurements of the anatomical structure produced by a measurement algorithm that processes the segmentation mesh; and
the search module is further configured to process the measurement data to produce the query response.

13. A system comprising:
the user interface system of any one of claims 1 to 12; and
a segmentation system configured to:
receive the medical image comprising the representation of the anatomical structure;
receive the initial segmentation mesh; and
reconfigure the initial segmentation mesh to the representation of the anatomical structure in the medical image using the model-based segmentation algorithm, to thereby produce the segmentation mesh.

14. A computer-implemented method for responding to a search query, the computer-implemented method comprising:
receiving metadata for a segmentation mesh of a representation of an anatomical structure in a medical image, wherein the metadata comprises parameter data generated by a model-based segmentation algorithm during production of the segmentation mesh by reconfiguration of an initial segmentation mesh to the representation of the anatomical structure;
converting the metadata into a text-based file containing one or more textual descriptors of the parameter data comprised in the metadata;
receiving the search query from a user;
processing at least the search query and the text-based file to produce a query response; and
controlling a user output interface to provide a user-perceptible output representing the query response to the user.

15. A computer program product comprising computer program code means which, when executed on a computing device having a processing system, cause the processing system to perform all of the steps of the computer-implemented method according to claim 14.
